# EUROPEAN PATENT SPECIFICATION

(11) **EP 2 432 440 B1**
(45) Date of publication and mention of the grant of the patent: **11.12.2013**
(21) Application number: 10732510.2
(22) Date of filing: 20.05.2010
(51) Int. Cl.: A61F 13/56, A61F 13/15, A61F 13/62

(54) **WEARABLE ABSORBENT ARTICLES WITH BONDED AND PRINTED FIBROUS MATERIALS**
TRAGBARE SAUGFÄHIGE ARTIKEL MIT GEKLEBTEN UND BEDRUCKTEN FASERMATERIALIEN
ARTICLES ABSORBANTS POUVANT ÊTRE PORTÉS AVEC MATIÈRES FIBREUSES LIÉES ET IMPRIMÉES

(30) Priority: 20.05.2009 US 180040 P; 07.07.2009 US 223477 P
(43) Date of publication of application: 28.03.2012
(73) Proprietor: The Procter & Gamble Company, Cincinnati, OH 45202 (US)
(72) Inventor: HORN, Thomas, Alexander, 65719 Hofheim (DE); ASHRAF, Arman, Mason Ohio 45040 (US); SAUER, Andrew, James, Cincinnati Ohio 45251 (US); MALDONADO, Clarissa, Cincinnati Ohio 45240 (US); TURNER, Robert, Haines, Cincinnati Ohio 45220 (US)
(74) Representative: Heide, Ute
(86) International application number: PCT/US2010/035542
(87) International publication number: WO 2010/135508

(56) References cited:
- WO-A2-02/22183
- WO-A2-02/098337
- US-A1- 2006 080 810
- US-A1- 2006 182 927
- US-A1- 2007 250 406
- US-A1- 2008 004 585

## Description

### FIELD

In general, embodiments of the present disclosure relate to wearable absorbent articles. In particular, embodiments of the present disclosure relate to wearable absorbent articles with bonded and printed fibrous materials.

### BACKGROUND

Wearable absorbent articles are useful for receiving, containing, and absorbing bodily exudates. Many front-fastenable wearable absorbent articles include bonded and printed fibrous materials. For example, such articles can include a hook and loop fastening system made from bonded and printed materials. In such systems, hook portions are provided on fastening ears and a loop portion is formed on the front of the article, so that the back of the article can be secured to the front.
U.S. Patent No. 2006/0080810 A1 discloses a knitted fabric female fastening portion for a mechanical fastener. The knitted fabric female fastening portion has a knitted fabric, an underlying substrate and a bonding layer. The bonding layer has a first plurality of non-intersecting bond lines and a second plurality of non-intersecting bond lines which are combined to form a pattern of intersecting bond lines that define tessellating pattern elements.
U.S. Patent No. 2007/0250406 A1 is related to a method of providing an interactive product system which includes selecting a plurality of character images. Each character image is associated with a unique characteristic selected from a set of unique characteristics. Each product has at least one of the character images displayed thereon. The caregiver is prompted to interact with a child when the caregiver observes the at least one character images on the product.
U.S. Patent No. 2008/0004585 A1 refers to a disposable absorbent article to be worn about the lower torso of a wearer that facilitates an easy and intuitive change. The disposable article includes a serviceable indicium that facilitates fitting of the disposable absorbent article to the wearer.
International Patent No. WO 02/22183 A2 discloses that an article to be worn about a wearer includes features that glow in the dark. These features are illuminative, are light emitting, or are reflective. These features may assist in the identification, location, entertainment, or changing of the wearer, as well as assist in the location of a fresh diaper for changing in a low light environment.
U.S. Patent No. 2009/068393 A1 is related to a laminated loop tape for a hook-and-loop fastener. The tape has a nonwoven textile support layer having an inner face, printing on the inner face, and at least partially transparent knitted cover layer having an inner face bearing on the support-layer inner face and an outer face provided with loops, and adhesive between the inner faces adhering the layers to each other.

A fibrous landing zone can be used to form the loop portion. It is often desirable to include printed graphics (such as letters, character images, and numbers) on the fibrous landing zone. However, for purposes of structural integrity and fastening performance, it is necessary to provide spaced apart bonded areas throughout the fibrous landing zone. Unfortunately, the presence of bonded areas on a fibrous landing zone makes it more difficult to visually discern printed graphics on the fibrous landing zone.

### BRIEF DESCRIPTION OF THE DRAWINGS

Figure 1 illustrates a perspective view of a front-fastenable wearable absorbent article formed for wearing, including a hook and loop fastening system with hook portions provided on fastening ears and a loop portion formed by a fibrous landing zone having printed graphics and a bond pattern, according to embodiments of the present disclosure.
Figure 2 illustrates a top view of an outside of a front-fastenable wearable absorbent article laid out in a flat and uncontracted state, including a hook and loop fastening system with hook portions provided on fastening ears and a loop portion formed by a nonwoven landing zone having printed graphics and a bond pattern, according to embodiments of the present disclosure.
Figure 3 illustrates a top view of an outside of a nonwoven landing zone with numerous randomly oriented nonwoven fibers, according to embodiments of the present disclosure.
Figure 4 illustrates a top view of an outside of a nonwoven landing zone with printed graphics, according to embodiments of the present disclosure.
Figure 5 illustrates a top view of an outside of a nonwoven landing zone with printed graphics and a bond pattern, according to embodiments of the present disclosure.
Figure 6 illustrates a top view of a bond pattern for a nonwoven landing zone, according to embodiments of the present disclosure.
Figure 7 illustrates a top view of an outside of a nonwoven landing zone with printed graphics and a bond pattern, according to embodiments of the present disclosure.
Figure 8A illustrates another view of the nonwoven landing zone with bond pattern from Figure 7.
Figure 8B illustrates the nonwoven landing zone of Figure 8A with the bond pattern over printed graphics.
Figure 9A illustrates a top view of a nonwoven landing zone with a modified version of the bond pattern of Figure 7, wherein the bond pattern has bond lines with a relatively shorter wavelength.
Figure 9B illustrates the nonwoven landing zone of Figure 9A with the bond pattern over printed graphics.
Figure 10A illustrates a top view of a nonwoven landing zone with a modified version of the bond pattern of Figure 7, wherein the bond pattern has bond lines with a relatively longer wavelength.
Figure 10B illustrates the nonwoven landing zone of Figure 10A with the bond pattern over printed graphics.
Figure 11A illustrates a top view of a nonwoven landing zone with a modified version of the bond pattern of Figure 7, wherein the bond pattern has a relatively smaller pitch and the bond lines have a relatively smaller amplitude.
Figure 11B illustrates the nonwoven landing zone of Figure 11A with the bond pattern over printed graphics.
Figure 12A illustrates a top view of a nonwoven landing zone with a modified version of the bond pattern of Figure 7, wherein the bond pattern has a relatively greater pitch and the bond lines have a relatively greater amplitude.
Figure 12B illustrates the nonwoven landing zone of. Figure 12A with the bond pattern over printed graphics.

### SUMMARY

The present disclosure includes front-fastenable wearable absorbent articles with fibrous landing zones having areas printed with graphics and bond patterns overlaying the printed areas, wherein the printed areas and the bond patterns are configured to allow the graphics to be readily visibly discernible.
A disposable wearable absorbent article comprises a non woven landing zone characterized in that the article includes a hook and loop fastening system with hook portions provided on fastening ears and a loop portion is formed by the nonwoven landing zone. The nonwoven landing zone includes: a graphic image, at least partially surrounded by a contour line having a contour line width. A bond pattern has a plurality of non-intersecting wavy bond lines overlaying the graphic image, wherein each of the bond lines has a bond line width; wherein the contour line width is greater than or equal to each of the bond line widths and wherein each of the bond line widths is greater than or equal to 0.2 millimeters and less than or equal to 2.0 millimeters.
The contour line has a uniform width (154).
The contour line width is at least 20% greater than each of the bond line widths.
The contour line width is at least 50% greater than each of the bond line widths.
The graphic image is completely surrounded by the contour line.
The landing zone includes a printed area and the contour line is formed by an unprinted portion.
The graphic image is a character image.
Each of the bond line widths is greater than or equal to 0.4 millimeters and less than or equal to 1.5 millimeters.
Each of the bond line widths is greater than or equal to 0.6 millimeters and less than or equal to 1.0 millimeters.

### DETAILED DESCRIPTION

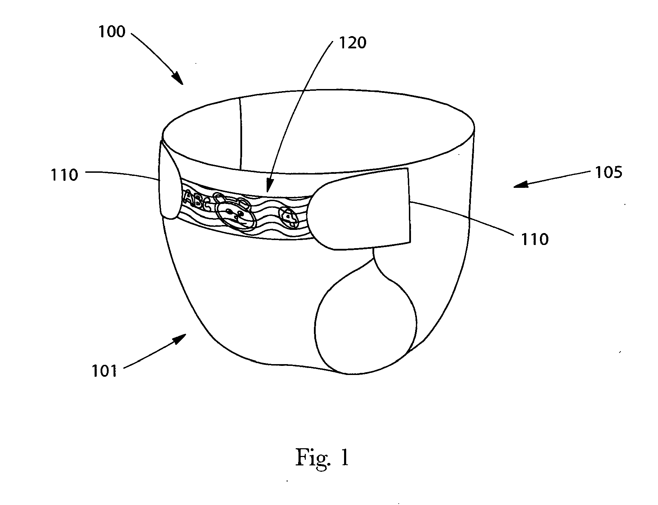

Figure 1 illustrates a perspective view of a front-fastenable wearable absorbent article 100 formed for wearing. The article 100 can include a topsheet, an absorbent core, and a backsheet. The article 100 is configured to receive, contain, and absorb bodily exudates. The article 100 includes a front 101 and a back 105. The article also includes a hook and loop fastening system with hook portions provided on fastening ears 110 and a loop portion formed by a fibrous landing zone 120. The hook portions on the fastening ears 110 can fasten to loops on the fibrous landing zone 120, so that the back 105 is secured to the front 101. The fibrous landing zone 120 has printed graphics and a bond pattern, according to embodiments of the present disclosure.

Throughout the present disclosure, landing zones are used to describe and illustrate various embodiments. However, embodiments of the present disclosure which are not limited to landing zones, but can be similarly applied to other bonded and printed fibrous materials in wearable absorbent articles are not in the scope of the present invention. For example, part, or parts, or all of a topsheet, a backsheet, a side ear, a side panel, a waistband, and/or a leg band of a wearable absorbent article could be configured with printed graphics and a bond patterns, but they are not in the scope of the present invention.

In embodiments throughout the present disclosure, a fibrous landing zone can be constructed of any fibrous materials in any manner known in the art. Fibrous materials can be made from animal fibers, plant fibers, mineral fibers, synthetic fibers, etc. Fibrous materials can include short fibers, long fibers, continuous fibers, fibers of varying lengths or cross-sectional geometries, or combinations of any of these. Throughout the present disclosure, nonwoven materials arc used to describe and illustrate various embodiments of fibrous materials, However, it is contemplated that embodiments of the present disclosure arc not limited to nonwoven material, but can be similarly applied to a wide variety of fibrous materials, such as those described above, as will be understood by one of skill in the art.

The term "nonwoven material" refers to a sheet-like structure (e.g. web) of fibers (sometimes referred to as filaments) that are interlaid in a non-uniform, irregular, or random manner. A nonwoven material can be a single layer structure or a multiple layer structure. A nonwoven material can also be joined to another material, such as a film, to form a laminate. A nonwoven material can be made from various natural and/or synthetic materials. Exemplary natural materials include cellulosic fibers, such as cotton, jute, pulp, and the like; and also can include reprocessed cellulosic fibers like rayon or viscose. Natural fibers for a nonwoven material can be prepared using various processes such as carding, etc. Exemplary synthetic materials include but are not limited to synthetic thermoplastic polymers that are known to form fibers, which include, but are not limited to, polyolefins, e.g., polyethylene, polypropylene, polybutylene and the like; polyamides, e.g., nylon 6, nylon 6/6, nylon 10, nylon 12 and the like; polyesters, e.g., polyethylene terephthalate, polybutylene terephthalate, polylactic acid and the like; polycarbonate; polystyrene; thermoplastic elastomers; vinyl polymers; polyurethane; and blends and copolymers thereof.

Also, in embodiments throughout the present disclosure, graphics can be printed on a nonwoven landing zone in any manner known in the art. As a first example, graphics can be printed on the fibers on the outside surface of the nonwoven layer of the landing zone; that is, the surface that is exposed, for receiving the fastening ears. As a second example, graphics can be printed on the fibers on the inside surface of the nonwoven layer of the landing zone; that is, the surface that is opposite from the outside surface. As a third example, graphics can be printed on an underlying substrate; that is, a substrate that lies under the nonwoven layer of the landing zone and thus faces the inside surface. Graphics can also be printed on a nonwoven landing zone using various combinations of any of these methods, or any other method known in the art.

Further, in embodiments throughout the present disclosure, a bond pattern can be applied to a nonwoven landing zone in any manner known in the art. As examples, a bond pattern can be applied by using heat, pressure, ultrasonic bonding, adhesive, other bonding means known in the art, or combinations of any of these. For instance, a nonwoven web can be bonded by passing the nonwoven web through a nip formed by a heated calendar roll (with a plurality of raised lands) and another roll, such that the lands form bond areas on the nonwoven web.

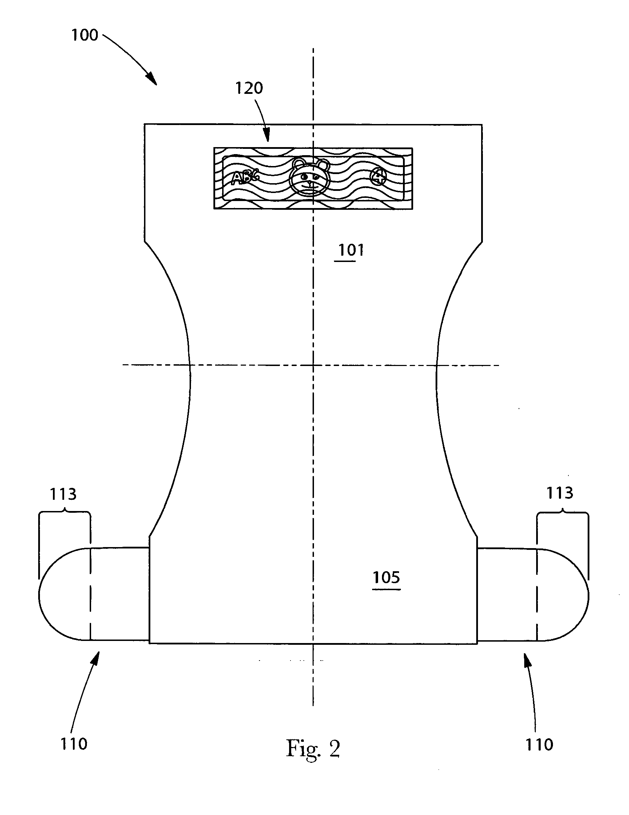

Figure 2 illustrates a top view of an outside of the front-fastenable wearable absorbent article 100 of Figure 1, laid out in a flat and uncontracted state. Each of the fastening ears 110 include a hook portion 115 provided on the distal end of the ear 110. However, in alternate embodiments, hook portions can be provided on other portions of an ear. The article includes a longitudinal centerline that divides the article 100 into left and right halves and a lateral centerline that divides the article 100 into the front 101 and the back 105. The fibrous landing zone 120, which is a nonwoven landing zone, is disposed in the front 101 of the article 101 with its width oriented laterally and its length oriented longitudinally. However, in alternate embodiments, a wearable absorbent article can be configured to be rear-fastenable, with a landing zone disposed in a back of the article. In various embodiments, a nonwoven landing zone can be formed from one or more discrete pieces of material or can be formed by one or more portion of elements of a wearable absorbent article, such a portion of an outer nonwoven layer of a backsheet.

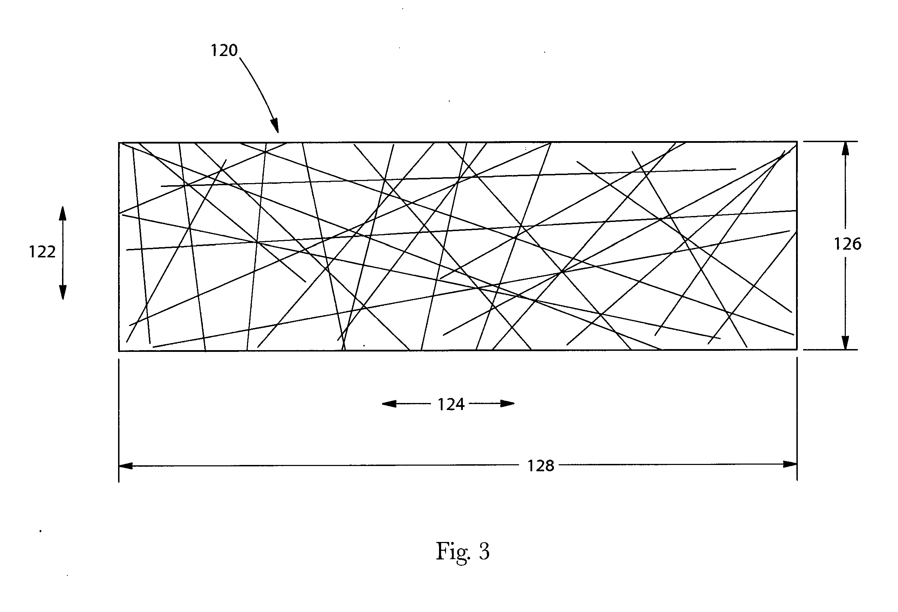

Figure 3 illustrates a top view of an outside of the nonwoven landing zone 120 of Figure 2. The landing zone 120 is a rectangular shaped piece of nonwoven material with numerous randomly oriented nonwoven fibers. However, in alternate embodiments, a landing zone can have various shapes, such as a curved shape or a chevron shape. The nonwoven material can be configured in numerous ways, as will be understood by one of ordinary skill in the art. For example, the nonwoven material can be a spunbond polypropylene material having substantially continuous fibers of 1.7 to 2.7 denier. The fibers in the nonwoven material form loops on the nonwoven landing zone 120, and the hooks of the fastening ears 110 can hook onto these loops.

The nonwoven web can be of different levels of opacity depending on the type of fibers, resins, and or the basis weight of the nonwoven used to make a nonwoven landing zone (opacity is typically measured by a Reflectance Spectrophotometer at 45 degrees). Opacity levels for nonwovens are known in the art to be sensitive, but not limited to the material density, thickness, and the degree of pigmentation used (Titaniumdioxide, TiO2). For example a high degree of nonwoven web opacity indicates a high degree of obscuring the background or an artwork behind it. Most common for hygiene nonwovens is the use of TiO2 at different levels for desired fiber pigmentation.

The landing zone 120 has a machine direction (MD) 122 and a cross direction (CD) 126, which are defined by the process for manufacturing the nonwoven material. In various embodiments, the fibers of the nonwoven material may or may not be configured to have an MD or CD directionality. The landing zone also has an overall longitudinal length 126 and an overall lateral width 128.

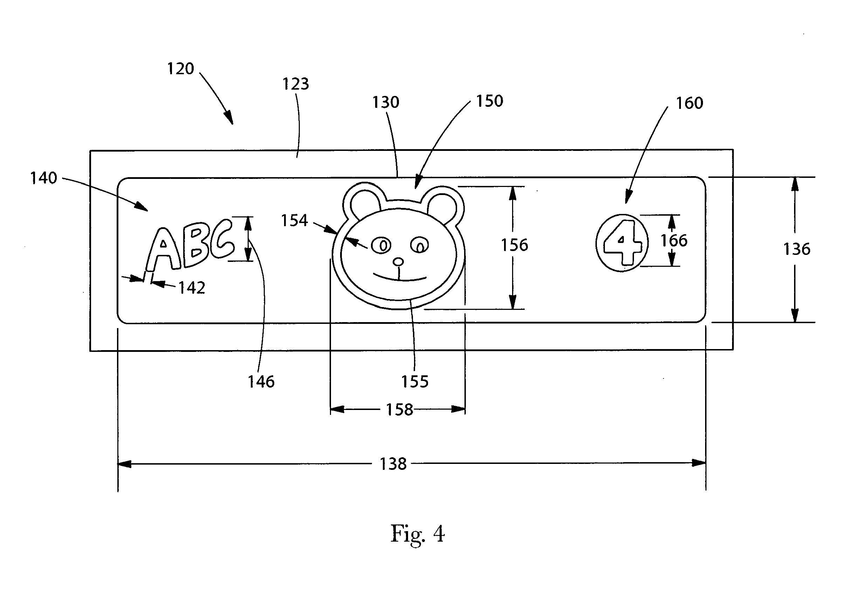

Figure 4 illustrates a top view of an outside of the nonwoven landing zone 120 of Figure 3 with printed graphics. For clarity, the nonwoven fibers are not shown in Figure 4. The landing zone 120 includes an unprinted border 123, which surrounds a printed area 130. However, in some embodiments, the nonwoven landing zone 120 may not include an unprinted border, such that the printed area coincides with the overall size and shape of the nonwoven landing zone. In an alternate embodiment, the printed area may overlap one or more outer edges of the nonwoven landing zone. In another alternate embodiment, the printed area may overlap all of the outer edges of the nonwoven landing zone.

The printed area 130 has an overall shape that is rectangular, although in various embodiments, the printed area can have an overall shape that is a different shape. The printed area 130 has an overall longitudinal length 136 and an overall lateral width 138. The printed area 130 includes various graphic images, such as letters 140, a character image 150, and a number 160. As used herein a graphic image can also include other recognizable images (such as a plant, a car, etc.) and geometric shapes, such as circles, squares, rectangles, triangles, stars, etc. A printed area of a nonwoven landing zone can also include other graphics of one or more colors. In the embodiment of Figure 4, the nonwoven landing zone 120 is reverse-printed; that is, ink is applied to the fibers on the inside surface of the nonwoven layer of the landing zone 120 (the bottom surface facing away from the viewer in Figure 4). In this embodiment, a nonwoven landing zone opacity can make it even more challenging to discern the backside printed graphics from the overall bonded areas of the nonwoven loop member because the overall artwork visibility is decreased with increasing opacity of the unprinted nonwoven web. In this case color differences as described below become even more important to increase visibility of artworks for consumers.

However, the nonwoven landing 120 can be printed on the outside surface or in various alternate ways, as will be understood by one of ordinary skill in the art.

The printed area 130 can include a background color and the character image 150 can include a character color. A background color surrounds at least a substantial portion of a character image and may or may not completely surround the character image. A printed area can include other colors in addition to a background color. In various embodiments, a background color can partially, or substantially, or completely fill a printed area. A character color fills at least a substantial portion of a character image and may or may not completely fill the character image. A character image can include other colors in addition to a character color.

In various embodiments, a background color can be different from, similar to, or the same as a character color. Tables 1, 2, and 3, below, provide six exemplary embodiments of Figure 4 with the nonwoven landing zone 120, the printed area 130, and the character image 150, wherein the character image 150 includes a character color and the printed area 130 includes a background color. Table 1 describes the background color, including CIELAB values, for each of the six embodiments. Table 2 describes the character color, including CIELAB values, for each of the six embodiments. Table 3 includes the differences in CIELAB values between the background color and the character color, for each of the six embodiments.

**Table 1**

| BACKGROUND COLOR | | | | |
|---|---|---|---|---|
| # | | L | A | b |
| 1 | Green | 69 | -25 | 57 |
| 2 | Orange | 54 | 51 | 24 |
| 3 | Yellow | 79 | 1 | 78 |
| 4 | Orange | 67 | 22 | 64 |
| 5 | Yellow | 79 | 1 | 78 |
| 6 | Orange | 54 | 51 | 24 |

**Table 2**

| CHARACTER COLOR | | | | |
|---|---|---|---|---|
| # | | L | a | b |
| 1 | pink | 66 | 33 | -2 |
| 2 | blue | 72 | -16 | -18 |
| 3 | blue | 77 | -12 | -12 |
| 4 | blue | 72 | -16 | -18 |
| 5 | blue | 72 | -16 | -18 |
| 6 | green | 74 | -18 | 28 |

**Table 3**

| DIFFERENCES | | | |
|---|---|---|---|
| # | L | a | b |
| 1 | 3 | 58 | 59 |
| 2 | 18 | 67 | 42 |
| 3 | 2 | 13 | 90 |
| 4 | 5 | 38 | 82 |
| 5 | 7 | 17 | 96 |
| 6 | 20 | 69 | 4 |

In the six examples described in Tables 1-3, colors can be obtained from Adobe Photoshop 10.0.1 (copyright 1990-2007, Adobe Systems Incorporated) using the Color Picker tool, which reports CIE L*a*b* colors based on the 1976 Commission Internationale d'Eclairage standard. For reference, Adobe Photoshop's CIE L*a*b* scales are 0 to 100 for L*, -128 to 127 for a*, and -128 to 127 for b*. The reported colors and ranges can represent target colors from an artwork file to be printed.

In these six examples, the differences in the lightness values between the background color and the character color ranged from 2-20. However, it is contemplated that a nonwoven landing zone with a printed area and a character image can have a background color and a character color with differences in CIELAB lightness (L) values of at least 10, at least 20, at least 30, at least 40, at least 50, or at least 60, or any integer value between these values. These differences in lightness can be determined by taking the absolute value of: the L value of the character color minus the L value of the background color. Without wishing to be bound by this theory, it is believed that differences in lightness between a background color and a character color allow a character image to be more readily seen in a printed area of a nonwoven landing zone.

In these six examples, the differences in the CIELAB a values between the background color and the character color ranged from 13-69. However, it is contemplated that a nonwoven landing zone with a printed area and a character image can have a background color and a character color with differences in CIELAB a values of at least 10, at least 20, at least 30, at least 40, at least 50, or at least 60, at least 70, at least 80, or any integer value between these values. These differences in a color can be determined by taking the absolute value of: the a value of the character color minus the a value of the background color. Also, in these six examples, the differences in the CIELAB b values between the background color and the character color ranged from 4-96. These differences in b color can be determined by taking the absolute value of: the b value of the character color minus the a value of the background color. However, it is contemplated that a nonwoven landing zone with a printed area and a character image can have a background color and a character color with differences in CIELAB b values of at least 10, at least 20, at least 30, at least 40, at least 50, or at least 60, at least 70, at least 80, at least 90, at least 100, or any integer value between these values.

Each of the letters 140 has a character width 142 and an overall height 146. All of the disclosure that relates to the character width of the letters can be similarly applied to the character width of numbers. The character image 150 has an overall height 156, measured longitudinally across the tallest portion of the character image, and an overall width 158, measured laterally across the widest portion of the character image. As used herein, the overall height and the overall width are considered major dimensions of the character image. The character image 150 is outlined by a contour line 155, which surrounds the character image 150, in order to highlight the character image 150 with respect to any background color or artwork in the printed area 130 and thus make the character image 150 more easily visible. In the embodiment of Figure 4, the contour line 155 completely surrounds the character image 150, however, in various embodiments, a contour line may only partially surround a character image. The contour line 155 has a uniform width 154, although contour line width may vary in some embodiments. In various embodiments, the contour line can be any printed color, such as white, grey, or black, or the contour line can be formed by an unprinted portion within the printed area. Also, in alternate embodiments, a contour line can similarly be applied to one or more letters, numbers, or other artwork images within a printed area. The number 160 has an overall height 166.

The dimensions of these printed graphics can be selected in relation to the dimensions of bond lines in a nonwoven bonding pattern applied to the nonwoven landing zone 120. In particular, the character width 142 of the letters 140, the width of the contour line 155, and the overall lengths, widths, and heights of images, letters, and numbers can be selected, in relation to dimensions of the bond lines, as described below.

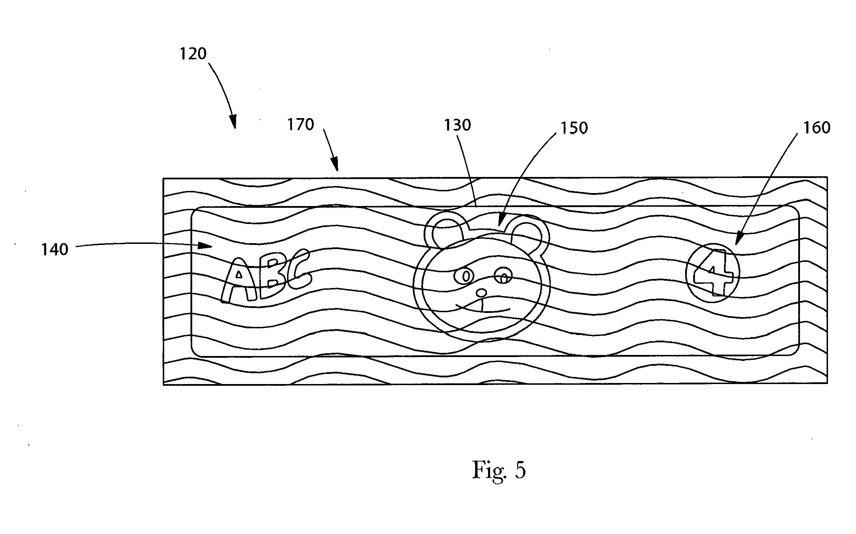

Figure 5 illustrates a top view of an outside of the nonwoven landing zone 120 of Figure 4 with the printed area 130, including the letters 140, the character image 150, and the number 160. The nonwoven landing zone 120 also includes a bond pattern 170 of wavy bond lines that extends over the entire nonwoven landing zone 120 and, as a result, overlays the entire printed area 130. For ease of illustration, throughout the present disclosure, the bond lines in the figures are illustrated as having no thickness, unless otherwise indicated. Although the bond pattern 170 overlays the printed area, the letters 140, the character image 150, and the number 160 are still readily visibly discernible due to selected dimensions in the printed graphics and the configuration of the bond pattern 170, as described herein. In some embodiments, a bond pattern may only extend over a portion of the nonwoven landing zone 120. The bond pattern 170 can be configured according to any embodiment of a bond pattern described in the present disclosure and/or according to any variation of any embodiment of a bond pattern described in the present disclosure.

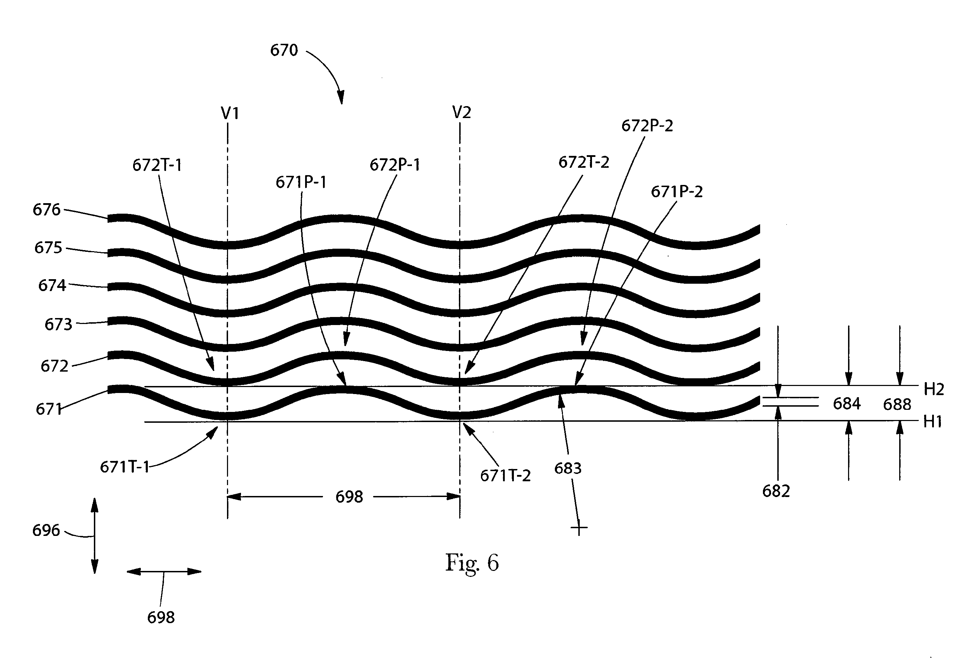

A bonded and printed fibrous material can be configured such that elements in the printed area, such as the overall width of a character image, the overall height of letters or numbers, the overall height of a character image, the contour width, and/or the character width, can be sized in relation to aspects of a bond pattern, such as the pitch of bond lines, the amplitude of bond lines, the wavelength of bond lines, and/or the width of bond lines, as described herein. The aspects of the bond pattern are defined in the description of the embodiment of Figure 6.

In the embodiment of Figure 5, the printed area 130 can be configured such that the overall width 158 of the character image 150 is at least 10% greater than a pitch of the bond pattern 170, or at least 20% greater than a pitch of the bond pattern 170, or at least 30% greater than a pitch of the bond pattern 170, or at least 40% greater than a pitch of the bond pattern 170, or at least 50% greater than a pitch of the bond pattern 170, or at least 75% greater than a pitch of the bond pattern 170, or at least 100% greater than a pitch of the bond pattern 170, or at least 150% greater than a pitch of the bond pattern 170, or at least 200% greater than a pitch of the bond pattern 170, or at least 300% greater than a pitch of the bond pattern 170, or at least 400% greater than a pitch of the bond pattern 170, or at least 500% greater than a pitch of the bond pattern 170, or at least 600% greater than a pitch of the bond pattern 170, or at least 700% greater than a pitch of the bond pattern 170, or at least 800% greater than a pitch of the bond pattern 170, or at least 900% greater than a pitch of the bond pattern 170. Alternatively, any of these relative size relationships can be applied to the amplitude of bond lines in a bond pattern, instead of being applied to the pitch. Also, alternatively, the pitch and/or amplitude of a bond pattern can be selected to create any of these ratios with respect to an overall width of one or more letters, character images, or numbers in a printed area. In other words, for a given printed area with images of particular sizes, the dimensions of the overlaying bond pattern can be adjusted to obtain the relationships described above. For example, if an overall width of a character is 10 millimeters, then a pitch of the bond pattern can be adjusted to be less than or equal to 50% (e.g. 5 millimeters). Without wishing to be bound by this theory, the present disclosure contemplates that letters, character images, or numbers with an overall width that is larger than a pitch and/or amplitude of a bond pattern will allow the printed image to be more readily visibly discernible from the bond pattern.

In the embodiment of Figure 5, the printed area 130 can be configured such that the overall height 146 of the letters 140 and/or the overall height 156 of the character image 150 and/or the overall height 166 of the number 160 is at least 10% greater than a pitch of the bond pattern 170, or at least 20% greater than a pitch of the bond pattern 170, or at least 30% greater than a pitch of the bond pattern 170, or at least 40% greater than a pitch of the bond pattern 170, or at least 50% greater than a pitch of the bond pattern 170, or at least 75% greater than a pitch of the bond pattern 170, or at least 100% greater than a pitch of the bond pattern 170, or at least 150% greater than a pitch of the bond pattern 170, or at least 200% greater than a pitch of the bond pattern 170, or at least 300% greater than a pitch of the bond pattern 170, or at least 400% greater than a pitch of the bond pattern 170, or at least 500% greater than a pitch of the bond pattern 170, or at least 600% greater than a pitch of the bond pattern 170, or at least 700% greater than a pitch of the bond pattern 170, or at least 800% greater than a pitch of the bond pattern 170, or at least 900% greater than a pitch of the bond pattern 170. Alternatively, any of these relative size relationships can be applied to the amplitude of bond lines in a bond pattern, instead of being applied to the pitch. Also, alternatively, the pitch and/or amplitude of a bond pattern can be selected to create any of these ratios with respect to an overall height of one or more letters, character images, or numbers in a printed area. In other words, for a given printed area with images of particular sizes, the dimensions of the overlaying bond pattern can be adjusted to obtain the relationships described above. For example, if an overall height of a character is 10 millimeters, then a pitch of the bond pattern can be adjusted to be less than or equal to 50% (e.g. 5 millimeters). Without wishing to be bound by this theory, the present disclosure contemplates that letters, character images, or numbers with an overall height that is larger than a pitch and/or amplitude of a bond pattern will allow the printed images to be more readily visibly discernible from the bond pattern.

In the embodiment of Figure 5, the printed area 130 can be configured such that the overall width 158 of the character image 150 is at least 10% greater than a wavelength of the bond pattern 170, or at least 20% greater than a wavelength of the bond pattern 170, or at least 30% greater than a wavelength of the bond pattern 170, or at least 40% greater than a wavelength of the bond pattern 170, or at least 50% greater than a wavelength of the bond pattern 170, or at least 75% greater than a wavelength of the bond pattern 170, or at least 100% greater than a wavelength of the bond pattern 170, or at least 150% greater than a wavelength of the bond pattern 170, or at least 200% greater than a wavelength of the bond pattern 170, or at least 300% greater than a wavelength of the bond pattern 170, or at least 400% greater than a wavelength of the bond pattern 170, or at least 500% greater than a wavelength of the bond pattern 170, or at least 600% greater than a wavelength of the bond pattern 170, or at least 700% greater than a wavelength of the bond pattern 170, or at least 800% greater than a wavelength of the bond pattern 170, or at least 900% greater than a wavelength of the bond pattern 170. Alternatively, the wavelength of a bond pattern can be selected to create any of these ratios with respect to an overall width of one or more letters, character images, or numbers in a printed area. Without wishing to be bound by this theory, the present disclosure contemplates that letters, character images, or numbers with an overall width that is larger than a wavelength of a bond pattern will allow the printed image to be more readily visibly discernible from the bond pattern.

In the embodiment of Figure 5, the printed area 130 can be configured such that the overall height 146 of the letters 140 and/or the overall height 156 of the character image 150 and/or the overall height 166 of the number 160 is at least 10% greater than a wavelength of the bond pattern 170, or at least 20% greater than a wavelength of the bond pattern 170, or at least 30% greater than a wavelength of the bond pattern 170, or at least 40% greater than a wavelength of the bond pattern 170, or at least 50% greater than a wavelength of the bond pattern 170, or at least 75% greater than a wavelength of the bond pattern 170, or at least 100% greater than a wavelength of the bond pattern 170, or at least 150% greater than a wavelength of the bond pattern 170, or at least 200% greater than a wavelength of the bond pattern 170, or at least 300% greater than a wavelength of the bond pattern 170, or at least 400% greater than a wavelength of the bond pattern 170, or at least 500% greater than a wavelength of the bond pattern 170, or at least 600% greater than a wavelength of the bond pattern 170, or at least 700% greater than a wavelength of the bond pattern 170, or at least 800% greater than a wavelength of the bond pattern 170, or at least 900% greater than a wavelength of the bond pattern 170. Alternatively, the wavelength of a bond pattern can be selected to create any of these ratios with respect to an overall height of one or more letters, character images, or numbers in a printed area. In other words, for a given printed area with images of particular sizes, the dimensions of the overlaying bond pattern can be adjusted to obtain the relationships described above. For example, if an overall height of a character is 10 millimeters, then a wavelength of the bond pattern can be adjusted to be less than or equal to 50% (e.g. 5 millimeters). Without wishing to be bound by this theory, the present disclosure contemplates that letters, character images, or numbers with an overall height that is larger than a wavelength of a bond pattern will allow the printed images to be more readily visibly discernible from the bond pattern.

In the embodiment of Figure 5, the printed area 130 can be configured such that the contour line width 154 of the contour line 155 and/or the character width 142 of the letters 140 is at least 10% greater than a bond line width of the bond pattern 170, or at least 20% greater than a bond line width of the bond pattern 170, or at least 30% greater than a bond line width of the bond pattern 170, or at least 40% greater than a bond line width of the bond pattern 170, or at least 50% greater than a bond line width of the bond pattern 170, or at least 75% greater than a bond line width of the bond pattern 170, or at least 100% greater than a bond line width of the bond pattern 170, or at least 150% greater than a bond line width of the bond pattern 170, or at least 200% greater than a bond line width of the bond pattern 170, or at least 300% greater than a bond line width of the bond pattern 170, or at least 400% greater than a bond line width of the bond pattern 170, or at least 500% greater than a bond line width of the bond pattern 170, or at least 600% greater than a bond line width of the bond pattern 170, or at least 700% greater than a bond line width of the bond pattern 170, or at least 800% greater than a bond line width of the bond pattern 170, or at least 900% greater than a bond line width of the bond pattern 170. Alternatively, the bond line width of a bond pattern can be selected to create any of these ratios with respect to a contour line width and/or a character width in a printed area. In other words, for a given printed area with images of particular sizes, the dimensions of the overlaying bond pattern can be adjusted to obtain the relationships described above. For example, if a width of a contour line is 2 millimeters, then a pitch of the bond pattern can be adjusted to be less than or equal to 50% (e.g. 1 millimeter). Without wishing to be bound by this theory, the present disclosure contemplates that contour line widths or character widths that are larger than a bond line width of a bond pattern will allow the printed images to be more readily visibly discernible from the bond pattern. Otherwise, if a bond line is narrower than a contour line width or a character width, the visibility of the printed images is impacted negatively as the wavy bond line becomes dominant and could be perceived as part of the printed images.

Figure 6 illustrates a top view of a portion of the bond pattern 670 for a nonwoven landing zone. The bond pattern 670 includes a longitudinal direction 696 and a lateral direction 698. The bond pattern 670 can be applied to a nonwoven landing zone such that the longitudinal direction 696 coincides with a machine direction of the nonwoven material of the landing zone and the lateral direction 698 coincides with a cross direction of the nonwoven material. In alternate embodiments, the bond pattern 670 can be angled, such that the longitudinal direction 696 is at an angle with respect to a machine direction of the nonwoven material of the landing zone. For example, a bond pattern may be angled at an angle of 0.26° with respect to a machine direction of the nonwoven material of the landing zone, in order to provide certain contact conditions between equipment and material during a bonding process, as will be understood by one of ordinary skill in the art.

The bond pattern 670 includes a plurality of bond lines 671, 672, 673, 674, 675, and 676. In Figure 6, the bond lines are illustrated as having thickness. The bond lines 671-676 can be thermal bonds or ultrasonic bonds or any other kind of bonds for bonding a nonwoven material. While each of the bond lines 671 through 676 is configured in the same way, this description will specifically refer to the details of bond lines 671 and 672 for ease of reference. In some embodiments, a bond pattern can include one or more bond lines that differ from the other bond lines in the pattern. The differing bond line(s) can differ in any way described herein. As used herein, the term "bond line" is intended to mean a recognizable pathway, formed by one or more bonded areas, and having a defined width that is substantially less than a defined length.

A bond pattern with a number of bond lines having a wavy shape is considered more useful than a bond pattern with a series of parallel bond lines having a straight line shape because straight lines would be unlikely to bond nonwoven fibers in some orientations. For example, a series of parallel bond lines oriented in the cross-direction on a nonwoven material would be unlikely to catch nonwoven fibers primarily oriented in the cross-direction and disposed between the bond lines. As a result, these unbonded fibers would offer reduced strength loops and thus a hook and loop fastening system with reduced performance. Thus, embodiments of the present disclosure contemplate bond patterns with a number of bond lines having a wavy shape. As used herein, the term "wavy" is intended to mean a non-linear shape that has at least some minimal variation in amplitude along its length; that is, a wavy shape deviates from its overall course by more than one thickness of its pathway. In various embodiments, a wavy shape can be continuously curved, can be partially curved and partially linear, or can be formed from a series of connected linear segments, such as a zig-zag pattern.

The bond line 671 is a continuous wavy line formed by a repeating pattern of identical waves. However, in various embodiments, a bond line can be a discontinuous line, or an effective line formed by a number of lines segments, or an effective line formed by a series of spaced apart bond areas. Also, in some embodiments, the waves may not be identical. The present disclosure contemplates that each wave in each bond line can vary in any way described herein.

In the bond pattern 670, the waves of each of the bond lines 671-676 are formed by an alternating series of peaks and troughs. In the illustrated portion of the pattern 670, the bond line 671 includes a first trough 671T-1 followed by a first peak 671P-1 followed by a second trough 671T-2 followed by a second peak 671P-2, and so on. The bond line 671 has a constant wave amplitude 684 measured longitudinally from top of peak to bottom of trough and a constant wavelength 686 measured laterally from a trough to an adjacent trough (or from a peak to an adjacent peak). In alternate embodiments, a bond line can have a varying wave amplitude and/or wavelength. The bond line 671 is continuously curved with an inside radius 683 on each peak and trough. In the embodiment of Figure 6, each of the other bond lines 672 through 676 also has the constant wave amplitude 684, the constant wavelength 686, and the inside radius 683.

The bond line 672 is configured in the same way as the bond line 671. Thus, the bond line 672 includes a first trough 672T-1, a first peak 672P-1, a second trough 672T-2, and a second peak 671P-2. The bond line 672 also has the constant amplitude 684 and the constant wavelength 686. In the lateral direction, the bond line 672 is in phase with the bond line 671, such that the peaks of the bond line 672 are aligned with the peaks of the bond line 671. The troughs of the bond lines 671 and 672 are similarly aligned.

Line V1 is a longitudinally oriented reference line drawn through the bottom of the first trough on each of the bond lines 671 through 676. Line V2 is a longitudinally oriented reference line drawn through the second trough on each of the bond lines 671 through 676. Since the bond lines 671 and 672 are in phase, the first trough 671T-1 and the first trough 672T-1 are both aligned on line V1. Since the bond lines 671 and 672 have the same wavelength 686, the second troughs 671T-2 and 672T-2 are aligned on line V2. Each of the other bond lines 673 through 676 is aligned in the same way with bond lines 671 and 672, as well as with each other.

The bond line 671 has a constant bond line width 682. Each of the other bond lines 672 through 676 has the same constant bond width. However, in some embodiments, a bond line width can vary along a bond line or from one bond line to another.

The bond line 672 is longitudinally offset from the bond line 671 by a pitch 688 measured longitudinally from top of peak on bond line 671 to top of peak on bond line 672 (or bottom of trough to bottom of trough, respectively). Each of the other bond lines 673 through 676 is longitudinally offset from the adjacent bond line by the same pitch 688 to create a regular array of bond lines in the bond pattern 670. As a result, all of the bond lines 671-676 are non-intersecting with respect to each other. In some alternate embodiments, a bond pattern can include bond lines offset from each other with varying pitches.

Line H1 is a laterally oriented reference line drawn along the bottoms of the troughs on the bond line 671. Line H2 is a laterally oriented reference line drawn along the tops of the peaks on the bond line 671. In the embodiment of Figure 6, the pitch 688 is the same as the amplitude 684. As a result, line H2 is also drawn along the bottoms of the troughs on the bond line 672.

In a bond pattern, the bond line width, the wave amplitude, the wavelength, and the pitch together determine the percent area that is bonded (and the percent area that is unbonded).

In various embodiments, it may be beneficial to the visual appearance of a printed area overlaid with a bond pattern, to reduce the bond line width while maintaining a target percent bonded area. In order to do so, the pitch of the bond pattern can be reduced, to control the percent bonded area. Without wishing to be bound by this theory, the present disclosure contemplates that a bond pattern with a reduced bond line width and a reduced pitch offers a more homogeneous appearance that is visually pleasing and allows images on the printed area to be more readily visibly discernible from the bond pattern.

For example, a bond pattern can have a bond line width of less than 0.8 millimeters, but a reduced pitch, in order to maintain a bonded area of at least 20%. As another example, a bond pattern can have a bond line width of less than 0.6 millimeters, but a reduced pitch, in order to maintain a bonded area of at least 20%. As a further example, a bond pattern can have a bond line width of less than 0.4 millimeters, but a reduced pitch, in order to maintain a bonded area of at least 20%. As still a further example, a bond pattern can have a bond line width of less than 0.2 millimeters, but a reduced pitch, in order to maintain a bonded area of at least 20%. Alternatively, these examples can also be implemented with other target percent bonded areas, such as targets of 15% or 25%, or any integer value for a percent between these two values.

However, there are practical lower limits to bond line width and bond pattern pitch. If a bond line width is reduced to a very small dimension (e.g. to about 0.1 millimeter or less), then some bonding processes may begin tearing or cutting nonwoven fibers rather than properly bonding them. Also, if a pitch of a bond pattern is reduced to a very small dimension (e.g. to about 1 millimeter or less), then there may be insufficient unbonded nonwoven fibers for forming loops, resulting in a need for much larger (and more expensive hook areas) or resulting in a hook and loop fastening system with reduced performance. These considerations will be understood by one of ordinary skill in the art.

In a particular embodiment, a bond pattern can be configured in the same way as the bond pattern 670, with the following dimensions: a constant bond line width of 0.6 millimeters, a constant wave amplitude of 3.4 millimeters, a constant wavelength of 24.42 millimeters, and a constant pitch of 3.4 millimeters, resulting in a bonded area of approximately 18%. The present disclosure contemplates variations of this particular embodiment wherein any of the dimensions described above can be varied independent from the others. This particular embodiment can be varied as described below.

One or more bond lines in a bond pattern can have a constant bond line width that can be less than 0.2 millimeters, or 0.2 millimeters, or 0.3 millimeters, or 0.4 millimeters, or 0.5 millimeters, or 0.6 millimeters, or 0.7 millimeters, or 0.8 millimeters, or 0.9 millimeters, or 1.0 millimeters, or 1.1 millimeters, or 1.2 millimeters, or 1.3 millimeters, or 1.4 millimeters, or 1.5 millimeters, or 1.6 millimeters, or 1.7 millimeters, or 1.8 millimeters, or 1.9 millimeters, or 2.0 millimeters, or greater than 2.0 millimeters, or any width between any of these specific values. Alternatively, a bond line width can vary between any of the values described above.

One or more bond lines in a bond pattern can have a constant wave amplitude that can be less than 2.0 millimeters, or 2.0 millimeters, or 2.5 millimeters, or 3.0 millimeters, or 3.5 millimeters, or 4.0 millimeters, or 4.5 millimeters, or 5.0 millimeters, or 5.5 millimeters, or 6.0 millimeters, or 6.5 millimeters, or 7.0 millimeters, or greater than 7.0 millimeters, or any value between any of these specific values, in increments of 0.1 millimeters. Alternatively, a wave amplitude can vary between any of the values described above. In various embodiments, it may be beneficial to the visual appearance of a printed area overlaid with a bond pattern to have a wave amplitude that is low (e.g. less than 10 millimeters) but non-zero. Without wishing to be bound by this theory, the present disclosure contemplates that a bond pattern having a wave amplitude that is low is visually pleasing, but still avoids the difficulties presented by bond lines having a straight line shape, as discussed above.

One or more bond lines in a bond pattern can have a constant wavelength that can be less than 5 millimeters, or 5 millimeters, or 10 millimeters, or 15 millimeters, or 20 millimeters, or 25 millimeters, or 30 millimeters, or 35 millimeters, or 40 millimeters, or 45 millimeters, or 50 millimeters, or greater than 50 millimeters or any integer value in millimeters between any of these specific values. Alternatively, a wavelength can vary between any of the values described above.

A bond pattern can have bond lines at a constant pitch that can be less than 2.0 millimeters, or 2.0 millimeters, or 2.5 millimeters, or 3.0 millimeters, or 3.5 millimeters, or 4.0 millimeters, or 4.5 millimeters, or 5.0 millimeters, or 5.5 millimeters, or 6.0 millimeters, or 6.5 millimeters, or 7.0 millimeters, or greater than 7.0 millimeters or any value between any of these specific values, in increments of 0.1 millimeters. Alternatively, a bond pattern pitch can vary between any of the values described above.

A bond pattern can result in a bonded area that can be less than 10%, or 10%, or 15%, or 20%, or 30%, or greater than 30%, or any integer value in percent between any of these specific percentages.

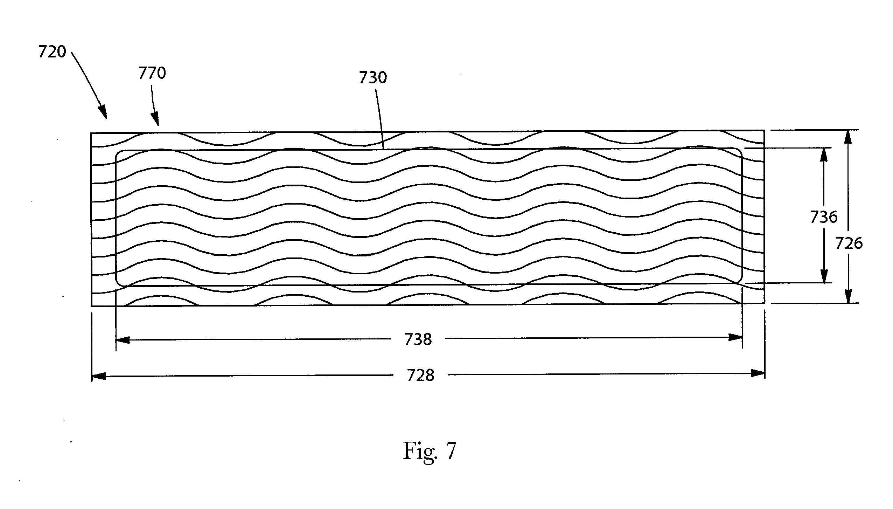

Figure 7 illustrates a top view of an outside of a nonwoven landing zone 720 with an overall longitudinal length 726 and an overall lateral width 728. The nonwoven landing zone 720 includes a printed area 730 with an overall longitudinal length 736 and an overall lateral width 738. The printed area 730 can be configured in the same way as the printed area 120 of Figure 5 or in any other way described herein. The nonwoven landing zone 720 also includes a bond pattern 770, which can be configured in the same way as any of the embodiments of the bond pattern 670 of Figure 6. In the embodiment of Figure 7, the bonding pattern 770 extends over the nonwoven landing zone 720 such that there are about five bond line wavelengths across the overall lateral width 728 and about ten bond lines over the overall longitudinal length 726.

In a particular embodiment, the nonwoven landing zone 720 can be configured with the following dimensions: an overall longitudinal length of the landing zone is 35 millimeters, an overall lateral width of the landing zone is 136 millimeters, an overall longitudinal length of the printed area is 25 millimeters, and an overall lateral width of the printed area is 126 millimeters. However, in various embodiments, any of these dimensions case be varied.

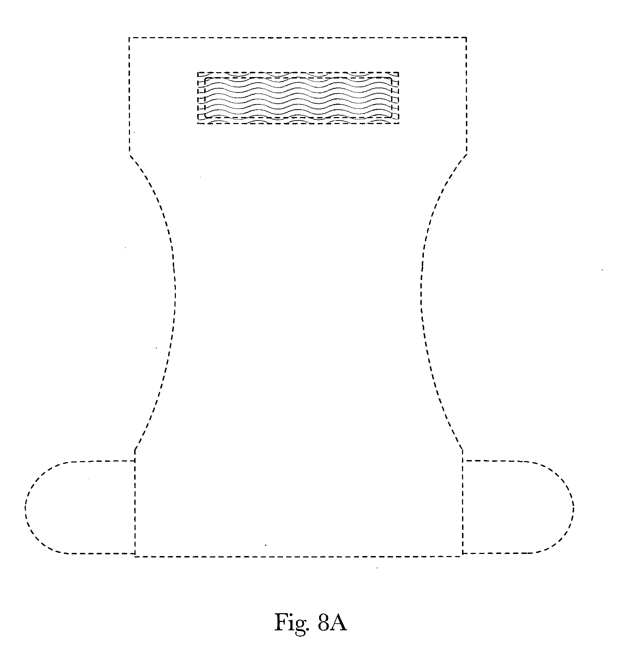

Figure 8A illustrates a top view of a front-fastenable wearable absorbent article, laid out in a flat and uncontracted state, having a nonwoven landing zone with the bond pattern from Figure 7. The present disclosure recognizes the article with the bond pattern of Figure 8A as an ornamental design, with the shape of the article, the printed area, and the outer edge of the nonwoven landing zone disclaimed, as indicated by the dashed lines.

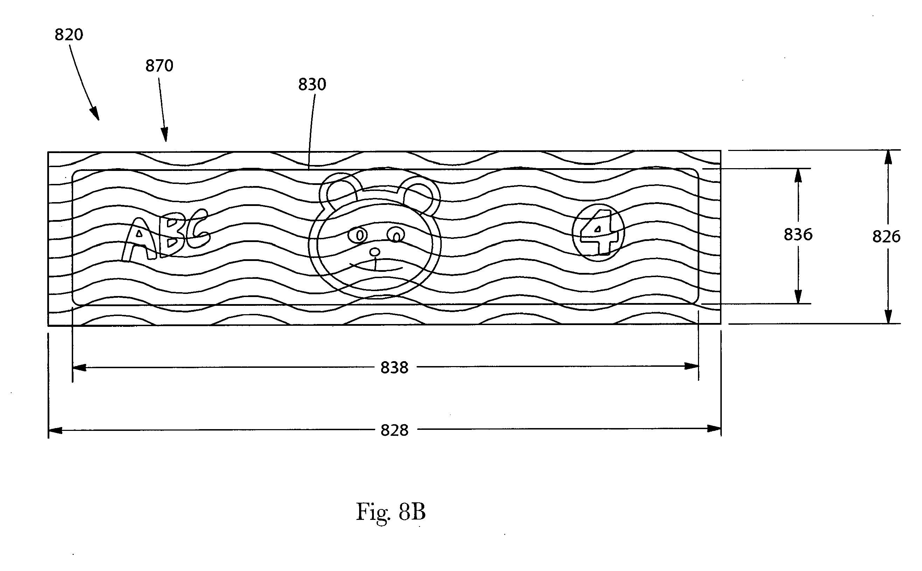

Figure 8B illustrates a top view of an outside of a nonwoven landing zone 820 with an overall longitudinal length 826 and an overall lateral width 828. The nonwoven landing zone 820 includes a printed area 830 with an overall longitudinal length 836 and an overall lateral width 838. The printed area 830 can be configured in the same way as the printed area 120 of Figure 5 or in any other way described herein. The nonwoven landing zone 820 also includes a bond pattern 870, which can be configured in the same way as the bond pattern 770 of Figure 7. In the embodiment of Figure 8B, the bonding pattern 870 extends over the nonwoven landing zone 820 such that there are about five bond line wavelengths across the overall lateral width 828 and about ten bond lines over the overall longitudinal length 826.

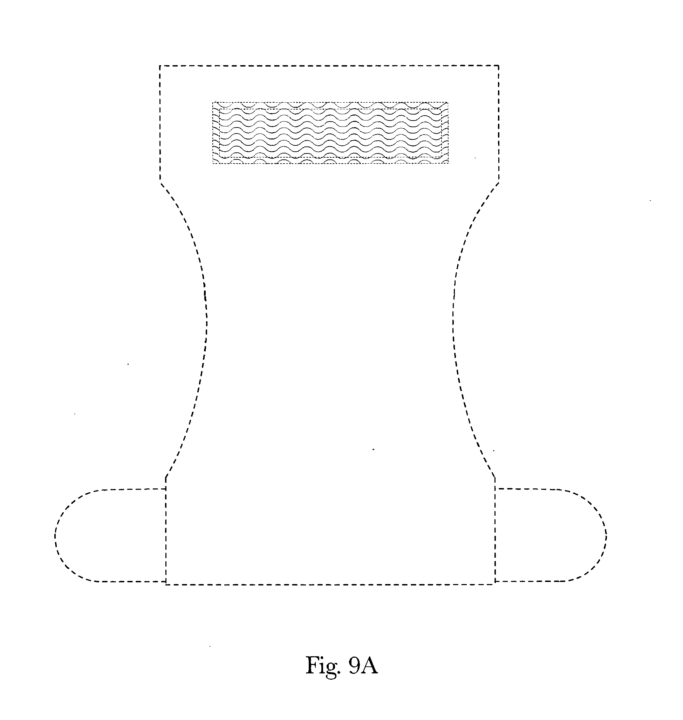

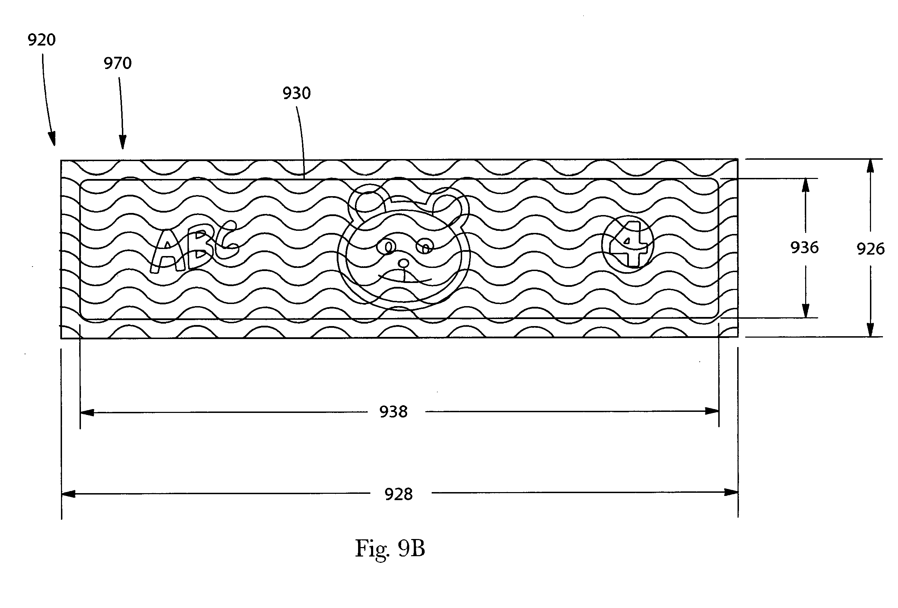

Figure 9A illustrates a top view of a front-fastenable wearable absorbent article, laid out in a flat and uncontracted state, having a nonwoven landing zone with the bond pattern of Figure 9B. The present disclosure recognizes the article with the bond pattern of Figure 9A as an ornamental design, with the shape of the article, the printed area, and the outer edge of the nonwoven landing zone disclaimed, as indicated by the dashed lines.

Figure 9B illustrates a top view of an outside of a nonwoven landing zone 920 with an overall longitudinal length 926 and an overall lateral width 928. The nonwoven landing zone 920 includes a printed area 930 with an overall longitudinal length 936 and an overall lateral width 938. The printed area 930 can be configured in the same way as the printed area 120 of Figure 5 or in any other way described herein. The nonwoven landing zone 920 also includes a bond pattern 970, which can be configured in the same way as the bond pattern 770 of Figure 7, except that the bond lines of the bond pattern 970 have a wavelength that is about half of the wavelength of the bond lines of the bond pattern 770. In the embodiment of Figure 9B, the bonding pattern 970 extends over the nonwoven landing zone 920 such that there are about ten bond line wavelengths across the overall lateral width 928 and about ten bond lines over the overall longitudinal length 926.

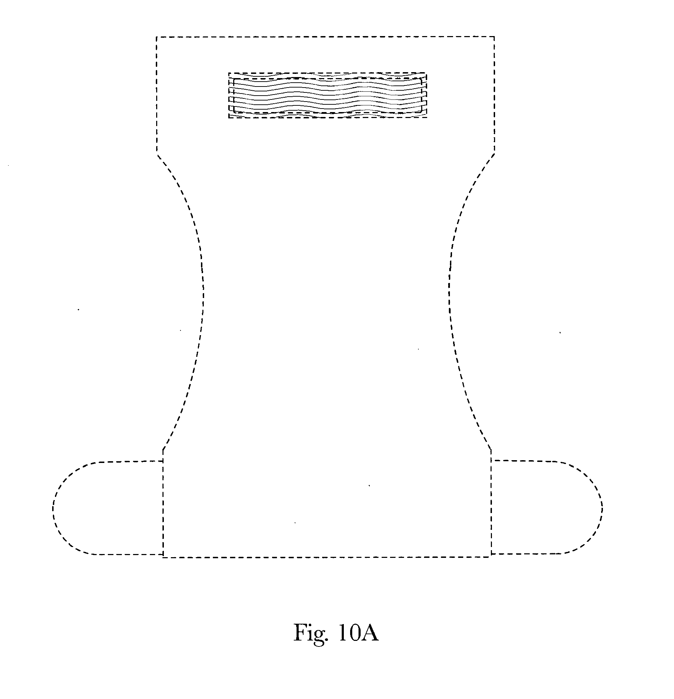

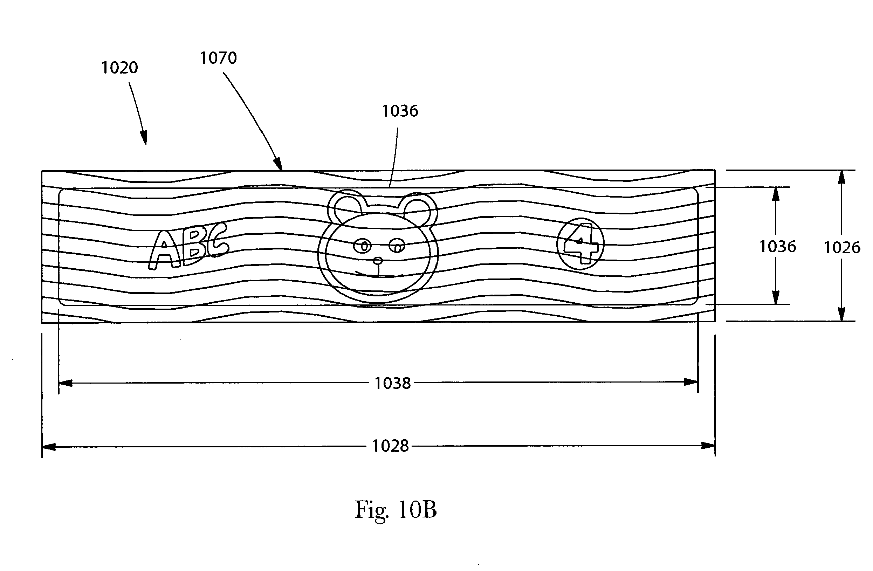

Figure 10A illustrates a top view of a front-fastenable wearable absorbent article, laid out in a flat and uncontracted state, having a nonwoven landing zone with the bond pattern of Figure 10B. The present disclosure recognizes the article with the bond pattern of Figure 10A as an ornamental design, with the shape of the article, the printed area, and the outer edge of the nonwoven landing zone disclaimed, as indicated by the dashed lines.

Figure 10B illustrates a top view of an outside of a nonwoven landing zone 1020 with an overall longitudinal length 1026 and an overall lateral width 1028. The nonwoven landing zone 1020 includes a printed area 1030 with an overall longitudinal length 1036 and an overall lateral width 1038. The printed area 1030 can be configured in the same way as the printed area 120 of Figure 5 or in any other way described herein. The nonwoven landing zone 1020 also includes a bond pattern 1070, which can be configured in the same way as the bond pattern 770 of Figure 7, except that the bond lines of the bond pattern 1070 have a wavelength that is about twice the wavelength of the bond lines of the bond pattern 770. In the embodiment of Figure 10B, the bonding pattern 1070 extends over the nonwoven landing zone 1020 such that there are about two and a half bond line wavelengths across the overall lateral width 1028 and about ten bond lines over the overall longitudinal length 1026.

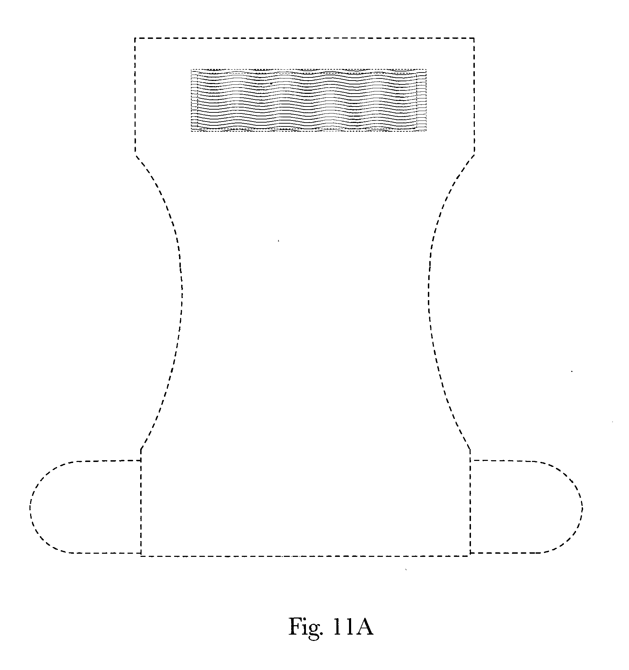

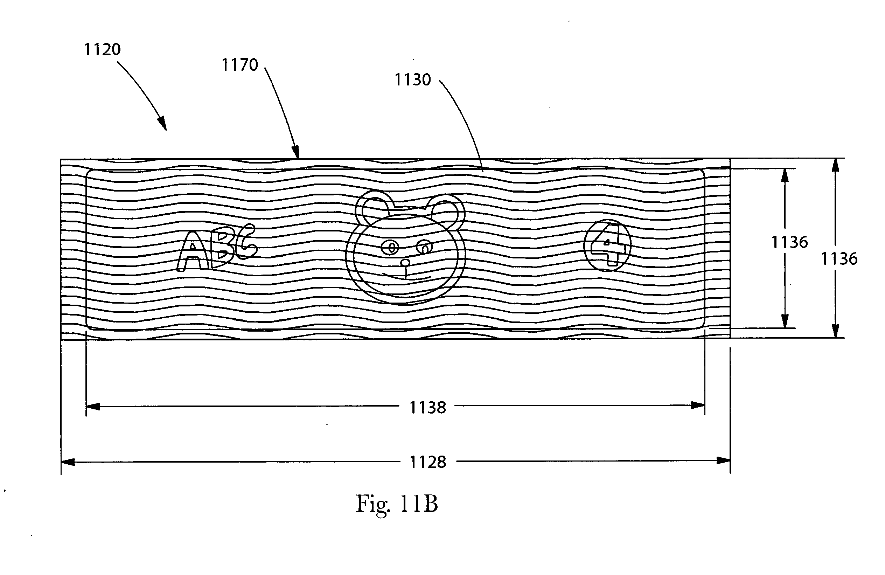

Figure 11A illustrates a top view of a front-fastenable wearable absorbent article, laid out in a flat and uncontracted state, having a nonwoven landing zone with the bond pattern of Figure 11B. The present disclosure recognizes the article with the bond pattern of Figure 11A as an ornamental design, with the shape of the article, the printed area, and the outer edge of the nonwoven landing zone disclaimed, as indicated by the dashed lines.

Figure 11B illustrates a top view of an outside of a nonwoven landing zone 1120 with an overall longitudinal length 1126 and an overall lateral width 1128. The nonwoven landing zone 1120 includes a printed area 1130 with an overall longitudinal length 1136 and an overall lateral width 1138. The printed area 1130 can be configured in the same way as the printed area 120 of Figure 5 or in any other way described herein. The nonwoven landing zone 1120 also includes a bond pattern 1170, which can be configured in the same way as the bond pattern 770 of Figure 7, except that the bond pattern 1170 has a pitch that is about half of the pitch of the bond pattern 770, and the bond lines of the bond pattern 1170 have an amplitude that is about half of the amplitude of the bond lines of the bond pattern 770. In the embodiment of Figure 11B, the bonding pattern 1170 extends over the nonwoven landing zone 1120 such that there are about five bond line wavelengths across the overall lateral width 1128 and about twenty bond lines over the overall longitudinal length 1126.

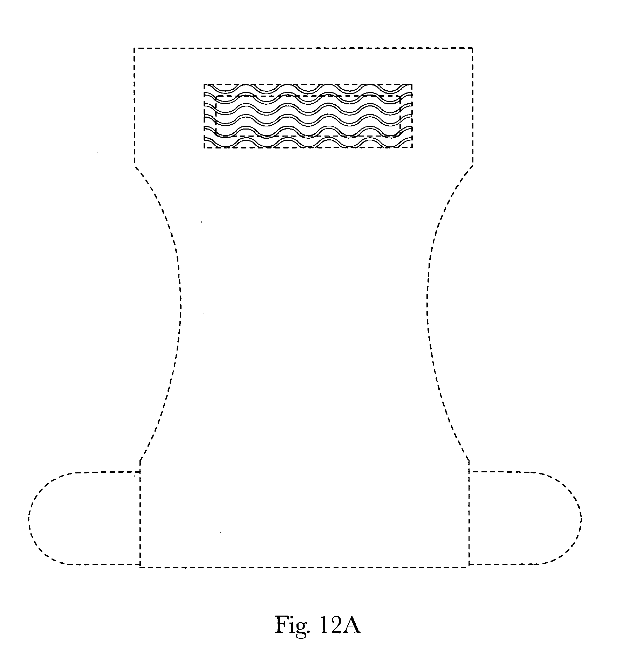

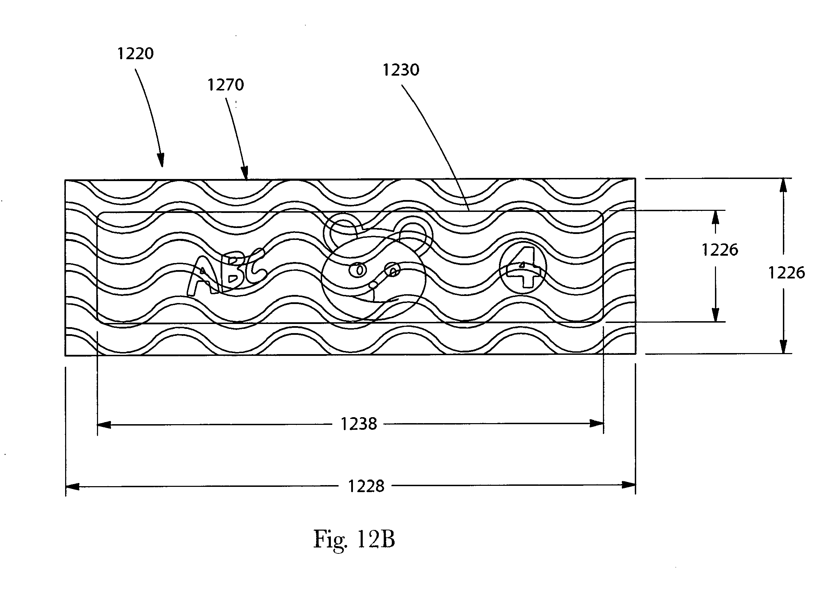

Figure 12A illustrates a top view of a front-fastenable wearable absorbent article, laid out in a flat and uncontracted state, having a nonwoven landing zone with the bond pattern of Figure 12B. In Figure 12A, the bond lines are illustrated as having thickness. The present disclosure recognizes the article with the bond pattern of Figure 12A as an ornamental design, with the shape of article, the printed area, and the outer edge of the nonwoven landing zone disclaimed, as indicated by the dashed lines.

Figure 12B illustrates a top view of an outside of a nonwoven landing zone 1220 with an overall longitudinal length 1226 and an overall lateral width 1228. The nonwoven landing zone 1220 includes a printed area 1230 with an overall longitudinal length 1236 and an overall lateral width 1238. The printed area 1230 can be configured in the same way as the printed area 120 of Figure 5 or in any other way described herein. The nonwoven landing zone 1220 also includes a bond pattern 1270, which can be configured in the same way as the bond pattern 770 of Figure 7, except that the bond pattern 1270 has a pitch that is about twice the pitch of the bond pattern 770, and the bond lines of the bond pattern 1270 have an amplitude that is about twice the amplitude of the bond lines of the bond pattern 770. In Figure 12B, the bond lines are illustrated as having thickness. In the embodiment of Figure 12B, the bonding pattern 1270 extends over the nonwoven landing zone 1220 such that there arc about five bond line wavelengths across the overall lateral width 1228 and about five bond lines over the overall longitudinal length 1226.

The dimensions and values disclosed herein arc not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A disposable wearable absorbent article (100), comprising a non woven landing zone (120), **characterized in that** the article (100) includes a hook and loop fastening system with hook portions provided on fastening ears (110) and a loop portion is formed by the nonwoven landing zone (120); and the nonwoven landing zone includes: a graphic image, at least partially surrounded by a contour line (155) having a contour line width (154); and a bond pattern (170) having a plurality of non-intersecting wavy bond lines overlaying the graphic image, wherein each of the bond lines has a bond line width; wherein the contour line width (154) is greater than or equal to each of the bond line widths and wherein each of the bond line widths is greater than or equal to 0.2 millimeters and less than or equal to 2.0 millimeters.

2. The disposable wearable absorbent article (100) of claim 1, **characterized in that** the contour line (155) has a uniform width (154).

3. The disposable wearable absorbent article (100) of claim 1 or 2, **characterized in that** the contour line width (154) is at least 20% greater than each of the bond line widths.

4. The disposable wearable absorbent article (100) of claim 1, 2, or 3, **characterized in that** the contour line width (154) is at least 50% greater than each of the bond line widths.

5. The disposable wearable absorbent article (100) of claim 1, 2, 3, or 4, **characterized in that** the graphic image is completely surrounded by the contour line (155).

6. The disposable wearable absorbent article (100) of claim 1, 2, 3, 4, or 5 **characterized in that** the landing zone (120) includes a printed area (130) and the contour line (155) is formed by an unprinted portion.

7. The disposable wearable absorbent article (100) of claim 1, 2, 3, 4, 5, or 6 **characterized in that** the graphic image is a character image (150).

8. The disposable wearable absorbent article (100) of claim 1, 2, 3, 4, 5, 6, or 7, **characterized in that** each of the bond line widths is greater than or equal to 0.4 millimeters and less than or equal to 1.5 millimeters.

9. The disposable wearable absorbent article (100) of claim 1, 2, 3, 4, 5, 6, or 7, **characterized in that** each of the bond line widths is greater than or equal to 0.6 millimeters and less than or equal to 1.0 millimeters.

## Patentansprüche

1. Einwegabsorptionsartikel (100) zum Anziehen, umfassend einen Anlegebereich (120) aus Vliesstoff, **dadurch gekennzeichnet, dass** der Artikel (100) einen Klettverschluss mit Hakenabschnitten, die auf Befestigungsflügeln (110) bereitgestellt sind, und einen Schlaufenabschnitt, der durch den Anlegebereich (120) aus Vliesstoff gebildet wird, beinhaltet; und wobei der Anlegebereich aus Vliesstoff Folgendes beinhaltet: ein Grafikbild, das mindestens teilweise von einer Konturlinie (155) umgeben ist, die eine Konturlinienbreite (154) aufweist; und ein Bindungsmuster (170), das eine Vielzahl sich nicht überschneidender wellenförmiger Bindungslinien, die das Grafikbild überlagern, aufweist, wobei jede der Bindungslinien eine Bindungslinienbreite aufweist; wobei die Konturlinienbreite (154) größer als oder gleich jeder der Bindungslinienbreiten ist und wobei jede der Bindungslinienbreiten größer als oder gleich 0,2 Millimeter und kleiner als oder gleich 2,0 Millimeter ist.

2. Einwegabsorptionsartikel (100) zum Anziehen nach Anspruch 1, **dadurch gekennzeichnet, dass** die Konturlinie (155) eine gleichmäßige Breite (154) aufweist.

3. Einwegabsorptionsartikel (100) zum Anziehen nach Anspruch 1 oder 2, **dadurch gekennzeichnet, dass** die Konturlinienbreite (154) mindestens 20 % größer als jede der Bindungslinienbreiten ist.

4. Einwegabsorptionsartikel (100) zum Anziehen nach Anspruch 1, 2 oder 3, **dadurch gekennzeichnet, dass** die Konturlinienbreite (154) mindestens 50 % größer als jede der Bindungslinienbreiten ist.

5. Einwegabsorptionsartikel (100) zum Anziehen nach Anspruch 1, 2, 3 oder 4, **dadurch gekennzeichnet, dass** das Grafikbild vollständig von der Konturlinie (155) umgeben ist.

6. Einwegabsorptionsartikel (100) zum Anziehen nach Anspruch 1, 2, 3, 4 oder 5, **dadurch gekennzeichnet, dass** der Anlegebereich (120) einen bedruckten Bereich (130) beinhaltet und die Konturlinie (155) von einem nicht bedruckten Abschnitt gebildet wird.

7. Einwegabsorptionsartikel (100) zum Anziehen nach Anspruch 1, 2, 3, 4, 5 oder 6, **dadurch gekennzeichnet, dass** das Grafikbild eine Zeichendarstellung (150) ist.

8. Einwegabsorptionsartikel (100) zum Anziehen nach Anspruch 1, 2, 3, 4, 5, 6 oder 7, **dadurch gekennzeichnet, dass** jede der Bindungslinienbreiten größer als oder gleich 0,4 Millimeter und kleiner als oder gleich 1,5 Millimeter ist.

9. Einwegabsorptionsartikel (100) zum Anziehen nach Anspruch 1, 2, 3, 4, 5, 6 oder 7, **dadurch gekennzeichnet, dass** jede der Bindungslinienbreiten größer als oder gleich 0,6 Millimeter und kleiner als oder gleich 1,0 Millimeter ist.

## Revendications

1. Article absorbant jetable pouvant être porté (100), comprenant une zone de réception non tissée (120), **caractérisé en ce que** l'article (100) inclut un système de fixation à crochet et boucle avec des parties de crochet fournies sur des pattes de fixation (110) et une partie de boucle est formée par la zone de réception non tissée (120) ; et la zone de réception non tissée inclut : une image graphique, au moins partiellement entourée par une ligne de contour (155) ayant une largeur de ligne de contour (154) ; et un motif de liaison (170) ayant une pluralité de lignes de liaison ondulées qui ne se croisent pas recouvrant l'image graphique, dans lequel chacune des lignes de liaison a une largeur de ligne de liaison ; dans lequel la largeur de ligne de contour (154) est supérieure ou égale à chacune des largeurs de ligne de liaison et dans lequel chacune des largeurs de ligne de liaison est supérieure ou égale à 0,2 millimètre et inférieure ou égale à 2,0 millimètres.

2. Article absorbant jetable pouvant être porté (100) selon la revendication 1, **caractérisé en ce que** la ligne de contour (155) a une largeur uniforme (154).

3. Article absorbant jetable pouvant être porté (100) selon la revendication 1 ou 2, **caractérisé en ce que** la largeur de ligne de contour (154) est d'au moins 20 % plus grande que chacune des largeurs de ligne de liaison.

4. Article absorbant jetable pouvant être porté (100) selon la revendication 1, 2 ou 3, **caractérisé en ce que** la largeur de ligne de contour (154) est d'au moins 50 % plus grande que chacune des largeurs de ligne de liaison.

5. Article absorbant jetable pouvant être porté (100) selon la revendication 1, 2, 3 ou 4, **caractérisé en ce que** l'image graphique est complètement entourée par la ligne de contour (155).

6. Article absorbant jetable pouvant être porté (100) selon la revendication 1, 2, 3, 4 ou 5, **caractérisé en ce que** la zone de réception (120) inclut une zone imprimée (130) et la ligne de contour (155) est formée par une partie non imprimée.

7. Article absorbant jetable pouvant être porté (100) selon la revendication 1, 2, 3, 4, 5 ou 6, **caractérisé en ce que** l'image graphique est une image de personnage (150).

8. Article absorbant jetable pouvant être porté (100) selon la revendication 1, 2, 3, 4, 5, 6 ou 7, **caractérisé en ce que** chacune des largeurs de ligne de liaison est supérieure ou égale à 0,4 millimètre et inférieure ou égale à 1,5 millimètre.

9. Article absorbant jetable pouvant être porté (100) selon la revendication 1, 2, 3, 4, 5, 6 ou 7, **caractérisé en ce que** chacune des largeurs de ligne de liaison est supérieure ou égale à 0,6 millimètre et inférieure ou égale à 1,0 millimètre.
